# EUROPEAN PATENT SPECIFICATION

(11) **EP 1 552 883 B1**
(45) Date of publication and mention of the grant of the patent: **27.08.2008**
(21) Application number: 03763910.1
(22) Date of filing: 10.07.2003
(51) Int. Cl.: B01J 29/70, C01B 39/48, C07C 2/66, C07C 15/085

(54) **Alkylation of benzene with propylene using a zeolite catalyst**
Die Alkylierung von Benzol mit Propylen mit einem Zeolithkatalysator
L'Alkylation du benzène à l'aide de propylène avec un catalyseur zéolitique

(30) Priority: 11.07.2002 ES 200201677
(43) Date of publication of application: 13.07.2005
(60) Divisional application: 08160770.7
(73) Proprietor: CONSEJO SUPERIOR DE INVESTIGACIONES CIENTIFICAS, 28006 Madrid (ES); UNIVERSIDAD POLITECNICA DE VALENCIA, 46022 Valencia (ES)
(72) Inventor: CORMA CANOS, A., Instituto de Tecnologia Quimica, C/Los Naranjos 46022 Valencia, ES (ES); REY GARCIA,F., 46022 Valencia (ES); DIAZ CABANAS,M.J., Instituto de tecnologia quimiqa, C/Los Naranjos 46022,ES (ES)
(74) Representative: Ungria Lopez, Javier
(86) International application number: PCT/ES2003/000351
(87) International publication number: WO 2004/007072

(56) References cited:
- EP-A- 0 940 178
- WO-A-99/08984
- WO-A-02/092511

## Description

### TECHNICAL FIELD

The present invention relates to the field of heterogeneous catalysis.

### BACKGROUND

Cumene is a product of commercial interest that is used as raw material in the production of phenol and acetone. Numerous processes have been developed using acid catalysts. A general reference on the catalysts and processes used can be found in "Encyclopedia of Chemical Processing and Design", J.J. McKezta and W.A. Cunningham Editors, V. 14, pp. 33-55 (1982). The alkylation of benzene with propylene, in addition to seeking a high conversion of propylene and a high selectivity for the monoalkylated product, isopropylbenzene (cumene), requires a minimum amount of n-propylbenzene (NPB) be formed. This is because NPB interferes with the oxidation of cumene to produce phenol and acetone, and consequently there is a need for a stream of cumene with the least amount of NPB impurities possible. Since it is difficult to separate cumene from NPB by conventional methods, such as distillation for example, then, logically, the yield of NPB must be as low as possible, and at any rate very low, during the alkylation of benzene with propylene.

From the viewpoint of the catalysts used in this process, acids such as H₃PO₄, AlCl₃ and HCl have conventionally been used, although they do give rise to problems arising from corrosion and loss of selectivity owing to the formation of polyalkylated products. Zeolites have also been used as catalysts for the alkylation of aromatics; thus, for example, patent US 4 292 457 describes zeolite ZSM-5 as a catalyst for the alkylation of benzene with propylene.

However, probably owing to the small diameter of its channels, this zeolite proves to be not very selective for the desired process. There are also many patents that describe the use of Faujasite and modified Faujasites as catalysts for the production of cumene by alkylation of benzene with propylene. More specifically, zeolite Y has good activity at temperatures of between 130 and 180°C, with good selectivity for the desired products. However, this selectivity decreases sharply when the conversion of benzene is increased, and it is therefore necessary to work with high benzene/propylene ratios in the feed. This results in high benzene recycling costs. Zeolite Beta has also been claimed as a catalyst for the alkylation of benzene with propylene in various patents, such as US 4 891 458, US 5 030 786, EP 432 814, EP 439 632, and EP 629 599. This zeolite produces good results in terms of activity and selectivity, but its Performance can be improved as regards both selectivity for NPB and the stability of the catalyst.
Spanish patent application P200101134 describes a zeolite material called ITQ21. However, it does not disclose a process for the alkylation of aromatic products with olefins and alcohols or polyalkylated aromatics in which this zeolite is used as catalyst. Spanish application P200102287, also, relates to said crystalline solid material specifically to its use in cracking, but it does not describe an alkylation process according to the present invention.

The subject of the present invention is a process for the alkylation of (benzene with propylene in the presence of a catalyst with an X-ray pattern and a chemical composition corresponding to ITQ-21, this catalyst being not active but also, in the case of cumene, producing a very low yield of n-propylbenzene, an unwanted product.

EP-0940178 discloses an alkylation procces of benzene with propylene using a surface modified zeolite beta. However, an improvement of the results shown in said document is needed.

### DESCRIPTION OF THE INVENTION

The present invention related to a process for preparing cymene, which prosses comprises an alkylation reaction of benzene with propylene in the presence of a catalyst which is a porous crystalline material with a chemical composition as express in its calcined form as:

X₂O₃:nYO₂:mZO₂

in which
(n+m) is at least 5,
X is a trivalent element,
Z is Ge,
Y is at least one tetravalent element other than Ge, and the n/m ratio is at least 1,
said material having, in its calcined form, an X-ray diffraction pattern whose most characteristic diffraction peaks are:

**Table 1**

| d(±0.3 Ä) | Relative Intensity |
|---|---|
| 13.64 | vs |
| 7.87 | vs |
| 4.82 | w |
| 4.55 | m |
| 4.11 | m |
| 3.41 | m |

in which d represents the interplanar spacing in ångström and the relative intensity of the lines is calculated as a percentage of the most intense peak, with
vs being a very strong relative intensity of 80-100,
m being a medium relative intensity of 40-60, and
w being a weak intensity of 20-40.

The trivalent elements incorporated are preferably Al, B, G, Fe, or mixtures thereof and the tetravalent elements are, for example, Si, Ge, Ti, or mixtures thereof, preferably Si and/or Ge.

The catalyst used in the alkylation process of the present invention can be prepared by hydrothermal synthesis from a mixture containing a source of one or more tetravalent elements, for example tetraethyl orthosilicate, amorphous silica, or silica with a mesoporous structure with or without long-range order (non-limiting examples) in the case of Si, or in the case of Ge the source can be germanium oxide, etc., optionally a source of one or more trivalent elements, for example aluminum alkoxides, alumina or metal aluminum (non-limiting examples) in the case where said trivalent element is Al, an organic structure directing agent (OSDA), water, and, in some cases, a source of fluoride ions, such as HF or NH₄F, among others. As structure directing agent a salt with an N(16)-methylsparteinium cation or the corresponding hydroxide can be used.

The reaction mixture has the following composition in terms of oxide molar ratios:

| | **Molar Ratio** | |
|---|---|---|
| **Reagents** | **Useable** | **Preferred** |
| (YO₂+ZO₂)/X₂O₃ | greater than 5 | greater than 7 |
| H₂O/(YO₂+ZO₂) | 1-50 | 2-20 |
| R/(YO₂+ZO₂) | 0.1-3.0 | 0.1-1.0 |
| YO₂/ZO₂ | greater than 1 | greater than 5 |

If fluoride anions are used, the ratio of this component in the synthesis mixture is as follows:

| | **Preferred** | **Useable** |
|---|---|---|
| F/(YO₂+ZO₂) | 0.1-3.0 | 0.1-1.0 |

The resulting mixture is placed in a steel autoclave with a Teflon lining and is heated at the desired temperature (between 80 and 200°C) for a time of between 12 hours and 30 days. The contents of the autoclave are filtered, washed and dried. The organic material in the solid obtained is removed by methods known in the literature, such as: calcination in the presence of N₂ followed by calcination in air, direct calcination in air, extraction of the organic material with inorganic or organic acids or by a treatment with ozone. The resulting material has an X-ray diffraction pattern in which at least the lines printed out in Table 1 are present.

The resulting catalyst is pelletized according to methods well known in the literature, using a diluent such as SiO₂, Al₂O₃, a clay, zirconia, magnesium oxide, or a mixture thereof, in zeolite/diluent proportions of between 20 and 95%, preferably between 40 and 90% by weight.

The catalyst can be treated with an aqueous solution of an inorganic acid, such as HNO₃, H₂SO₄, H₃PO₄ or HClO₄, at a pH of between 0 and 1.5, at temperatures of between 20 and 100°C, for a time of between 10 and 400 minutes, depending on the acid concentrations and the treatment temperature. The ratio of the catalyst to the acid aqueous solution is between 0.05 and 1, and preferably between 0.1 and 0.5 by weight.

Once calcined at a temperature of between 450 and 700°C, the resulting catalyst, with or without acid treatment, but containing acid centers, is used as catalyst in the alkylation of benzene with propylene.

In an alternate embodiment, the zeolite material in uncalcined form, or following heat treatment, can undergo balancing cation exchange, if said cations are present, in which said balancing cations are exchanged with other balancing cations such as metal ions, H⁺ and H⁺ precursors, such as NH⁺₄ for example. Among the cations that can be introduced by ion exchange, preference is given to those that can play a positive role in the activity of the material as a catalyst, more specifically cations such as H⁺, cations of rare earths and Group VIII metals, and metals of Groups IIA, IIIA, IVA, VA, IB, IIB, IIIB, IVB, VB, VIIB of the Periodic Table of the Elements. As examples of these cations, mention may be made of cations with an n+ charge, selected from among Na⁺, K⁺, Cs⁺, Ca²⁺, Mg²⁺, Ni²⁺, Cu²⁺ and Co²⁺.

According to the aromatics alkylation process of the present invention, the alkylation reaction takes place at a reaction temperature of between 60 and 350°C, preferably between 80 and 300°C. The pressure is between 1.4 and 7.0 MPa, preferably between 1.4 and 4.1 MPa. The space velocity (WHSV) of the reagents is between 0.2 and 10 hours⁻¹, preferably between 0.5 and 10 hours⁻¹. The aromatic compound/alkylating agent molar ratio is between 2 and 20, preferably between 2 and 15.

In the process of the present invention, the alkylated aromatic compound is cumene, the aromatic compound is benzene and the alkylating agent is propylene.

The alkylation reaction takes place at a reaction temperature of between 60 and 350°C, preferably between 80 and 300°C. The pressure is between 1.4 and 7.0 MPa, preferably between 1.4 and 4.1 MPa, the space velocity (WHSV) of reagents is between 0.2 and 10 hours⁻¹, preferably between 0.5 and 10 hours⁻¹, and the benzene/propylene molar ratio is between 2 and 20, preferably between 2 and 15.

According to an alternate embodiment of the present process, propylene is reacted with benzene under alkylation conditions, in which a liquid phase at least partially exists, in a molar ratio of between 2 and 20, in the presence of the catalyst.

According to the process of the present invention, the aromatic compound is benzene.

According to this invention - the acid catalysts prepared with the crystalline structure described - it has been found that, when applied in acid form to the alkylation of benzene with propylene, they are very active and have a surprisingly low selectivity for the production of NPB. Furthermore, the selectivity for cumene can be increased by introducing appropriate amounts of alkali metals, alkaline earth metals, or metal cations into the material, by ion exchange, as stated above. Its selectivity can also be increased by removing the surface acidity by extraction of trivalent cations in the network, such as Al and/or B for example, by means of a treatment with inorganic acids or other chemical agents that are capable of extracting said elements. Catalysts based on the cationic exchange or leaching treatments mentioned above make it possible to decrease selectivity for polyalkylated products.

The catalysts thus obtained and formulated with the diluents and proportions described above are used in the reaction systems and conditions described above for the alkylation of benzene with propylene.

A number of illustrative examples of the preparation of the catalyst and its use in the alkylation of benzene with propylene, are described below.

### EXAMPLES

### Example 1

This example illustrates the preparation of an acidic crystalline solid catalyst.

0.86 g of aluminum isopropoxide and 0.53 g of GeO₂ were dissolved in 34.42 g of N(16)-methylsparteinium hydroxide solution at a concentration of 1.53 mol/kg. 4.74 g of tetraethyl orthosilicate were hydrolyzed in the solution obtained, and stirring was maintained, allowing all the alcohol formed during hydrolysis to evaporate. 0.52 g of hydrofluoric acid solution (48.1% HF by weight) was then added. The final composition of the synthesis gel was:

0.95SiO₂:0.05GeO₂:0.02Al₂O₃:0.50ROH:0.50HF:2H₂O

in which ROH is N(16)-methylsparteinium hydroxide.

The gel was heated at 175°C for 5 days in steel autoclaves with a Teflon internal lining. The solid obtained was filtered, washed with distilled water, dried at 100°C and calcined in air at 580°C for 3 hours. The X-ray diffraction diagram for this material presents the peaks shown in Table 2.

**Table 2**

| d(±0.2) (Å) | Relative Intensity |
|---|---|
| 13.64 | vs |
| 9.64 | vw |
| 7.87 | vs |
| 6.82 | vw |
| 4.82 | w |
| 4.55 | m |
| 4.11 | m |
| 3.78 | vw |
| 3.41 | m |
| 3.31 | vw |
| 3.13 | vw |
| 3.05 | vw |
| 2.91 | vw |
| 2.67 | vw |
| 2.62 | vw |
| 2.53 | vw |
| 2.41 | vw |

### Example 2

This example illustrates the preparation of the catalyst in basic medium.

0.25 g of aluminum isopropoxide and 0.53 g of GeO₂ were dissolved in 26.58 g of N(16)-methylsparteinium hydroxide solution at a concentration of 0.56 mol/kg. 5.21 g of tetraethyl orthosilicate were hydrolyzed in the solution obtained, and stirring was maintained, allowing evaporation of all the alcohol formed during hydrolysis and of the water necessary until the final composition of the gel was:

0.83SiO₂:0.17GeO₂:0.02Al₂O₃:0.50ROH:6H₂O

in which ROH is N(16)-methylsparteinium hydroxide.

The gel was heated at 175°C for 20 days in steel autoclaves with a Teflon internal lining. The solid obtained was filtered, washed with distilled water, dried at 100°C and calcined in air at 580°C for 3 hours. The X-ray diffraction diagram for this material presents the peaks shown in Table 2.

### Example 3

The present example illustrates the use of a material prepared according to Example 1 as a catalyst in the alkylation of benzene with propylene.

A sample with an Si/Al ratio of 25, prepared according to Example 1, was pelletized, selecting a particle size between 0.25 and 0.42 mm to perform the reaction. The zeolite (0.55 g) was diluted with silicon carbide (0.59-0.84 mm) in an SiC/zeolite weight ratio of 5. The diluted catalyst was placed in a tubular steel reactor 1 cm in diameter, and 100 ml/min were passed through under standard N₂ conditions at 150°C for 1.5 hours. The temperature was then lowered to 20°C and the flow of N₂ was stopped. At this point, benzene (1200 µl/min) was fed in and the pressure was raised to 3.5 MPa. When the pressure of 3.5 MPa was reached, the temperature was raised to 125°C and the feed of propylene (270 µl/min) was begun, at a benzene/propylene molar ratio of 3.4.

The results in terms of converted propylene are shown in Table 3.

**Table 3: Conversion and selectivity in the alkylation of benzene with propylene at 125°C, B/P = 3.4 mol/mol, prop WHSV = 18 h⁻¹, P = 3.5 MPa, Si/Al ratio = 25**

| Reaction time (min) | Conversion (%) | Selectivity with respect to propylene (%) | | | |
|---|---|---|---|---|---|
| | | Cumene | DIPB | NPB | Other |
| 15 | 99.39 | 87.61 | 11.86 | 0.04 | 0.49 |
| 120 | 99.88 | 88.29 | 11.12 | 0.03 | 0.56 |
| 195 | 99.14 | 87.81 | 11.29 | 0.03 | 0.87 |
| 270 | 99.30 | 87.76 | 11.40 | 0.04 | 0.80 |

In this example, the NPB and diisopropylbenzene (DIPB) values are very low, lower than those obtained with a zeolite Beta with the same Si/Al ratio operating under the same reaction conditions.

### Example 4

The present example shows the influence of the space velocity (WHSV) (12 h⁻¹) on the conversion and selectivity for the alkylation of benzene with propylene using the same catalyst as in Example 3, the rest of the reaction conditions being the same as in Example 3.

The conversion results and reaction time are shown in Table 4.

**Table 4: Conversion and selectivity in the alkylation of benzene with propylene at 125°C, B/P = 3.4 mol/mol, prop WHSV = 12 h⁻¹, P = 3.5 MPa, Si/Al ratio = 25**

| Reaction time (min) | Conversion (%) | Selectivity with respect to propylene (%) | | | |
|---|---|---|---|---|---|
| | | Cumene | DIPB | NPB | Other |
| 90 | 99.16 | 81.65 | 16.47 | 0.02 | 0.32 |
| 225 | 98.70 | 86.96 | 12.16 | 0.02 | 0.86 |
| 345 | 98.20 | 85.42 | 13.70 | 0.03 | 0.84 |

## Claims

1. A process for preparing cumene, which process comprises an alkylation reaction of benzene with propylene in the presence of a catalyst which is a porous crystalline material with a chemical composition expresed in its calcined form as:
X₂O₃:nYO₂:mZO₂
in which
(n+m) is at least 5,
X is a trivalent element
Z is Ge,
Y is at least one tetravalent element other than Ge, and the n/m ratio is at least 1,
said material having, in its calcined form, an X-ray diffrection pattern whose most characteristic diffrection peaks are:
| d(±0.3 Å) | Relative Intensity |
|---|---|
| 13.64 | vs |
| 7.87 | vs |
| 4.82 | W |
| 4.55 | m |
| 4.11 | m |
| 3.41 | m |
in which d represents the interplanar spacing in angström and the relative intensity of the lines is calculated as a percentage of the most intense peak, with
vs being a very strong relative intensity of 80-100,
m being a medium relative intensity of 40-60, and
w being a weak intensity of 20-40.

2. The process as claimed in claim 1, wherein the alkylation reaction takes place at a reaction temperature of between 60 and 350°C, the pressure is between 1.4 and 7.0 Mpa, the space velocity (WHSV) of reagents is between 0.2 and 10 hours⁻¹, and the benzene/propylene molar ration is between 2 and 20.

3. The process as claimed in claim 1, wherein the porous crystalline material is subjected to cation exchange with cations selected from among H⁺, cations of rare earths, and cations of metals of Group VIII, IIA, IIIA, IVA, VA, IB, IIB, IIIB, IVB, VB and VIIB of the Periodic Table of the Elements.

## Patentansprüche

1. Verfahren zum Herstellen von Cumol, wobei das Verfahren eine Alkylierungsreaktion von Benzol mit Propylen in Gegenwart eines Katalysators umfasst, welcher ein poröses kristallines Material mit einer chemischen Zusammensetzung ist, die in ihrer kalzinierten Form wie folgt wiedergegeben wird:
X₂O₃:nYO₂:mZO₂
wobei
(n+m) wenigstens 5 ist,
X ein dreiwertiges Element ist,
Z Ge ist,
Y wenigstens ein vierwertiges Element ist, das von Ge verschieden ist, und das n/m-Verhältnis wenigstens 1 beträgt,
wobei das Material, in seiner kalzinierten Form, ein Röntgenbeugungsdiagramm aufweist, dessen am meisten charakteristische Beugungspeaks die folgenden sind:
| d (± 0,3 Å) | relative Intensität |
|---|---|
| 13,64 | vs |
| 7,87 | vs |
| 4,82 | W |
| 4,55 | m |
| 4,11 | m |
| 3,41 | m |
wobei d den Gitterebenenabstand in Angström wiedergibt und die relative Intensität der Linien als ein Prozentsatz des intensivsten Peaks berechnet ist, wobei
vs eine sehr starke relative Intensität von 80-100 ist,
m eine mittlere relative Intensität von 40-60 ist, und
w eine schwache Intensität von 20-40 ist.

2. Verfahren nach Anspruch 1, wobei die Alkylierungsreaktion bei einer Reaktionstemperatur zwischen 60 und 350 °C stattfindet, der Druck zwischen 1,4 und 7,0 MPa liegt, die Raumgeschwindigkeit (WHSV) der Reagenzien zwischen 0,2 und 10 Stunden⁻¹ liegt und das Benzol/Propylen-Molverhältnis zwischen 2 und 20 liegt.

3. Verfahren nach Anspruch 1, wobei das poröse kristalline Material einem Kationenaustausch mit Kationen unterworfen wird, die ausgewählt sind aus H⁺, Kationen von seltenen Erden und Kationen von Metallen der Gruppe VIII, IIA, IIIA, IVA, VA, IB, IIB, IIIB, IVB, VB und VIIB des Periodensystems der Elemente.

## Revendications

1. Procédé de préparation de cumène, lequel procédé comprend une réaction d'alkylation du benzène avec du propylène en présence d'un catalyseur qui est un matériau cristallin poreux ayant une composition chimique exprimée sous sa forme calcinée par :
X₂O₃ : nYO₂ : mZO₂
dans laquelle
(n+m) vaut au moins 5,
x est un élément trivalent,
Z est Ge,
Y est au moins un élément tétravalent autre que Ge, et le rapport n/m vaut au moins 1,
ledit matériau ayant, sous sa forme calcinée, un diagramme de diffraction de rayons X dont les pics de diffraction les plus caractéristiques sont :
| d(±0,3 Å) | Intensité relative |
|---|---|
| 13,64 | vs |
| 7,87 | vs |
| 4,82 | W |
| 4,55 | m |
| 4,11 | m |
| 3,41 | m |
dans lequel d représente l'espacement interplanaire en angström et l'intensité relative des lignes est calculée comme un pourcentage du pic le plus intense, avec
vs qui est une intensité relative très forte de 80 à 100,
m qui est une intensité relative moyenne de 40 à 60, et
w qui est une intensité faible de 20 à 40.

2. Procédé selon la revendication 1, dans lequel la réaction d'alkylation a lieu à une température de réaction comprise entre 60 et 350 °C, la pression est comprise entre 1,4 et 7,0 Mpa, la vitesse spatiale (WHSV) des réactifs est comprise entre 0,2 et 10 heures⁻¹ et le rapport molaire benzène/propylène est compris entre 2 et 20.

3. Procédé selon la revendication 1, dans lequel le matériau cristallin poreux est soumis à un échange cationique avec des cations choisis parmi H⁺, des cations de lanthanide et des cations de métaux des groupes VIII, IIA, IIIA, IVA, VA, IB, IIB, IIIB, IVB, VB et VIIB du tableau périodique des éléments.
